(19) Europäisches Patentamt
European Patent Office
Office européen des brevets

(11) **EP 2 314 200 B1**

(12) **EUROPÄISCHE PATENTSCHRIFT**

(45) Veröffentlichungstag und Bekanntmachung des Hinweises auf die Patenterteilung:
**13.01.2016 Patentblatt 2016/02**

(51) Int Cl.:
*A61B 3/107* (2006.01)     *G01B 11/25* (2006.01)

(21) Anmeldenummer: **09013294.5**

(22) Anmeldetag: **21.10.2009**

(54) **Vorrichtung und Verfahren zum Vermessen einer Cornea**

Method and device for measuring a cornea

Dispositif et procédé destinés à la mesure d'une cornée

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**27.04.2011 Patentblatt 2011/17**

(73) Patentinhaber: **SIS AG, Surgical Instrument Systems**
**2562 Port (CH)**

(72) Erfinder:
• **Rathjen, Christian**
**28197 Bremen (DE)**

• **Cattin, Roger**
**2552 Orpund (CH)**
• **Meier, Christoph**
**2560 Nidau (CH)**

(74) Vertreter: **Rentsch Partner AG**
**Rechtsanwälte und Patentanwälte**
**Fraumünsterstrasse 9**
**Postfach 2441**
**8022 Zürich (CH)**

(56) Entgegenhaltungen:
**EP-A2- 0 445 618        DE-A1- 4 007 502**
**DE-A1-102007 018 048    US-A- 5 054 907**
**US-A- 5 116 115         US-A- 5 592 246**

## Beschreibung

Technisches Gebiet

Die vorliegende Erfindung betrifft eine Vorrichtung und ein Verfahren zum Vermessen einer Cornea. Die vorliegende Erfindung betrifft insbesondere eine ophthalmologische Vorrichtung und ein ophthalmologisches Verfahren, in welchen zum Vermessen der Cornea ein zweidimensionales Referenzmuster auf die Cornea projiziert, und ein von der Cornea auf Grund des Referenzmusters reflektiertes Reflexionsmuster erfasst wird.

Stand der Technik

Keratometer (oder Ophthalmometer) werden zur Messung der Oberflächenkrümmung der Cornea (Hornhaut im Auge) und Bestimmung der Hornhautverläufe verwendet. Dabei wird die Reflexion eines beleuchteten Referenzobjekts auf der Hornhaut erfasst und darauf basierend die Krümmung der spiegelnden Corneaoberfläche bestimmt. Bei bekannten Videokeratometern wird eine Anzahl von konzentrischen Ringen auf die Cornea projiziert. Dabei wird bevorzugt ein diffus streuender Körper (Schirm) mit einem Muster (z.B. Placidomuster) versehen respektive beleuchtet, das vom Auge reflektiert und von einer zumeist auf der visuellen Achse des Auges befindlichen Kamera aufgenommen wird. Neben radialen Ringmustern gibt es auch andere diskrete Muster mit binären Hell-Dunkelstrukturen wie beispielsweise zweidimensionale schachbrettartige Muster. Aus der Deformation der Ringe auf der Cornea wird mit zusätzlichen Annahmen über den Abstand des Auges, d.h. über den Ort der Reflexion auf der Cornea, die Oberflächenneigung der Cornea ermittelt, welche Rückschlüsse auf die Brechkraft der Cornea ermöglicht. Basierend auf einer erwarteten geometrischen Ausgestaltung (Form) des Auges lässt sich auch eine dreidimensionale Gestalt der Cornea über die Integration der radialen Oberflächenneigung bestimmen. Aufgrund der geringen Anzahl der radialen Stützpunkte, d.h. der geringen Anzahl Ringe respektive Hell-Dunkelübergänge (typischerweise zwischen fünfzehn und zwanzig Ringe) sind diese Messverfahren respektive Messgeräte auf Grund ihrer begrenzten radialen Auflösung nicht sehr genau und fehlerbehaftet.

Zur Verbesserung der Genauigkeit und des Messbereich schlägt US 5,953,100 die Verwendung mehrerer Kameras vor, welche Reflexionen am Auge aus verschiedenen Perspektiven erfassen. Aufgrund der spiegelnden Reflexion des Auges kann allerdings ein korrespondierender Objektpunkt des Auges in den Ansichten der Kameras nicht ausgemacht werden (d.h. eine Triangulation ist nicht möglich). Daher muss jedes Bild einer Kameraansicht für sich ausgewertet werden und die Daten müssen in einem abschliessenden Schritt zusammengefügt werden. Solche Verfahren erhöhnen nur bedingt die Genauigkeit. Die Anzahl der Messpunkte erhöht sich maximal proportional zur Anzahl der verwendeten Kameras.

In der Patentschrift US 6,926,408 wird für die Bestimmung der Corneatopografie gemäss dem obenstehenden Verfahren ein kontinuierliches zweidimensionales Muster vorgeschlagen, welches ein sinusförmiges radiales Intensitätsprofil und ein sinusförmiges winkelabhängiges Farbprofil verwendet. Das Muster wird entweder als flache Form erzeugt und auf einer durchscheinenden Konusform angebracht oder die Farben werden direkt auf eine geeignete Oberfläche aufgetragen. Der Patient wird mit seinem Auge vor das zweidimensionale Muster positioniert, und das auf der Cornea reflektierte Reflexionsmuster wird mit einer CCD-Kamera (Charged Coupled Device) erfasst. Zur Bestimmung der Corneatopografie werden die Bildpunkte des erfassten Reflexionsmusters jeweils mit den entsprechenden Reflexionspunkten auf der Cornea korreliert. Durch die Verwendung eines kontinuierlichen Referenzmusters wird die Anzahl potentieller Messpunkte im Vergleich zu den eingangs erwähnten diskreten Mustern erhöht, was eine Verbesserung der Messgenauigkeit ermöglicht. Für eine robustere Bildverarbeitung schlägt US 6,926,408 die Anwendung von Bandpassfiltern vor, was jedoch die Auflösung reduziert, da abhängig vom Filter (insbesondere von dessen Zentralfrequenz und Bandbreite) jeweils nur ausgefilterte Teile des erfassten Reflexionsmusters ausgewertet werden, welche eine geringere Auflösung aufweisen als das ungefilterte Reflexionsmuster. Bandpassfilter funktionieren umso besser je kontinuierlicher das Signal und je geringer die Frequenzmodulation ist. Diese Bedingungen sind am Auge aufgrund der hohen patientenabhängigen Variabilität, d.h. der starken Verzerrung des Ringmusters, und unterbrochenen Ringen (z.B. durch Wimpern) nicht gegeben. Mit anderen Worten, die Verwendung eines kontinuierlichen Referenzmusters erhöht zwar die Auflösung im Vergleich zu diskreten Mustern, die Anwendung von Bandpassfiltern auf das entsprechende Reflexionsmuster führt aber nicht zu einer kontinuierlichen Messung, die jeden Bildpunkt (Pixel) für sich misst. Aufgrund der geringen Vorteile und der oben genannten praktischen Beschränkungen haben es Verfahren mit kontinuierlichen Mustern bisher nicht zur Produktreife gebracht. Verfahren, die jeden Bildpunkt für sich messen und damit eine maximale örtliche Auflösung bzw. Messpunktanzahl erzielen, welche eine Grössenordnung über bisherigen Verfahren liegt, existieren zurzeit nicht.

In US 5,116,115 werden ein Verfahren und eine Vorrichtung zum Messen der Corneatopografie beschrieben, in welchen ein phasenverschiebender Bildwerfer verwendet wird um ein sinusförmiges Intensitätsmuster in einer Beleuchtungsrichtung auf die Cornea zu projizieren. Gemäss US 5,116,115 wird die Cornea mit einer dünnen Folie bedeckt, welche darauf treffende Lichtstrahlen diffus reflektiert. Mittels einer herkömmlichen Videokamera werden reflektierte Lichtstrahlen aus einer von der Projektionsrichtung abweichenden Richtung erfasst, danach digitalisiert und in einem Computer ausgewertet. Basierend auf mindestens drei Intensitätsmessungen mit jeweils um 90° phasenverschobenen Intensitätsmustern wird für jeden Projektionspunkt ein Phasenwert und darauf basierend die Corneatopografie berechnet.

US 5,592,246 beschreibt eine Vorrichtung zum Erfassen der hinteren und vorderen Corneaoberfläche durch Projektion von Mustern mittels einer LCD Anzeige und Erfassen der Reflektionsmuster, wobei sequentiell mehrere unterschiedliche Muster projiziert und erfasst werden und dabei Augenbewegungen laufend kompensiert werden.

DE 10 2007 018 048 beschreibt ein hauptsächlich in der Mikroskopie angewandtes Verfahren zur Erzeugung von optischen Schnittbildern durch strukturierte Beleuchtung einer Probe mit bloss zwei, beispielsweise komplementären Lichtverteilungen. Dabei ist ein optischer Schnitt eine Abbildung, welche Objektdetails der Probe abbildet, die in der Fokusebene liegen, und Objektdetails unterdrückt, die nicht in der Fokusebene liegen. Gemäss DE 10 2007 018 048 wird mit Hilfe eines eingeschwenkten Kalibrierobjekst eine Kalibriermessung durchgeführt, um die durch optische Abbildungsfehler verursachten unterschiedlichen lokalen Phasen im Detektorkoordinatensystem zu bestimmen und dann bei der Erzeugung der optischen Schnittbildern zur Korrektur der optischen Abbildungsfehler zu verwenden.

Darstellung der Erfindung

Es ist eine Aufgabe der vorliegenden Erfindung eine Vorrichtung und ein Verfahren vorzuschlagen, welche zum Vermessen der Cornea ein zweidimensionales Referenzmuster auf die Cornea projizieren, ein von der Cornea auf Grund des Referenzmusters reflektiertes Reflexionsmuster erfassen und mindestens gewisse Nachteile des Stands der Technik überwinden. Es ist insbesondere eine Aufgabe der vorliegenden Erfindung eine ophthalmologische Vorrichtung und ein ophthalmologisches Verfahren vorzuschlagen, welche ein zweidimensionales Referenzmuster auf die Cornea projizieren, ein von der Cornea auf Grund des Referenzmusters reflektiertes Reflexionsmuster erfassen und eine kontinuierliche Vermessung der Cornea ermöglichen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die Vermessung der Cornea erfolgt erfindungsgemäß auf der Basis von mehreren unterschiedlichen Reflexionsbildern, welche jeweils aus derselben Perspektive von einem oder mehreren von der Cornea reflektierten Reflexionsmustern erfasst werden. Vorzugsweise wird ein periodisches Referenzmuster, beispielsweise mit einer radial verlaufenden Periode erzeugt.

In einer Variante wird das periodische Referenzmuster mit einer variierbaren Periode erzeugt. Die Referenzmuster werden beispielsweise durch Modulation von mindestens einem physikalischen Lichtparameter wie Lichtintensität, Lichtpolarisation und /oder Lichtwellenlänge erzeugt.

Das zweidimensionale Referenzmuster wird beispielsweise mit einer aktiven Anzeige, insbesondere mit einer LCD-Anzeige, auf die Cornea, oder mit einem Projektor (räumlicher Lichtmodulator oder Spatial Light Modulator) auf einen passiven Schirm projiziert, wobei diese Projektion von vorne oder bei einem als ausgestalteten transluzenten Schirm von hinten erfolgen kann. Mit dem Ausdruck "Projektion des zweidimensionalen Referenzmusters auf die Cornea" soll begrifflich sowohl die Projektion mittels der aktiven Anzeige als auch die Ausstrahlung über den passiven Schirm respektive den räumlichen Lichtmodulator mitumfasst sein.

Die Vermessung der Cornea basierend auf mehreren unterschiedlichen Reflexionsbildern von einem oder mehreren von der Cornea reflektierten Reflexionsmustern hat insbesondere den Vorteil, dass eine kontinuierliche Vermessung der Cornea ermöglicht wird, wobei für jeden Punkt auf der Cornea entsprechende Pixel (Bildpunkte) erfasst und ausgewertet werden, was im Vergleich zu bekannten Messverfahren eine wesentlich höhere Auflösung respektive höhere Anzahl von Messpunkten bei der Vermessung der Cornea ermöglicht.

Erfindungsgemäß wird für Punkte auf der Cornea jeweils ein Phasenwert des Reflexionsmusters basierend auf in den gespeicherten Reflexionsbildern jeweils am entsprechenden Bildpunkt gemessenen Intensitäten berechnet, und der mindestens eine geometrische Messwert der Cornea wird erfindungsgemäß basierend auf dem Phasenwert bestimmt. Beispielsweise wird für Punkte auf der Cornea basierend auf den Reflexionsbildern jeweils der Phasenwert des Reflexionsmusters am betreffenden Punkt durch Auswertung der untenstehenden Gleichung berechnet:

$$I(x', y') = I_0(x', y')\{1 + sm(x', y')\cos[\varphi(x', y')]\},$$

wobei $I(x', y')$ eine gemessene Intensität an einem dem betreffenden Punkt entsprechenden Bildpunkt mit den Koordinaten $x', y'$, $I_0(x', y')$ eine Hintergrundintensität (Summe aus Beleuchtungsmittelwert von Referenzmuster und Hintergrundlicht) am Bildpunkt, $sm(x', y')$ eine Signalmodulation am Bildpunkt, und $\varphi(x', y')$ der Phasenwert am Bildpunkt und somit am betreffenden Punkt auf der Cornea ist.

In einer Ausführungsvariante werden mehrere zueinander verschobene kontinuierliche, zweidimensionale Referenzmuster zur jeweiligen Projektion auf die Cornea erzeugt, und es wird jeweils ein Reflexionsbild von von der Cornea auf Grund der verschobenen Referenzmuster reflektierten Reflexionsmustern gespeichert. Die mehreren zueinander verschobenen Referenzmuster werden Erfindungsgemäß durch Phasenverschiebung eines periodischen Referenzgrundmusters erzeugt, und es wird jeweils ein Reflexionsbild von von der Cornea auf Grund der phasenverschobenen Referenzmuster reflektierten Reflexionsmustern gespeichert. Die Erzeugung mehrerer (phasen-)verschobener Referenzmuster und die Erfassung der entsprechenden Reflexionsbilder ermöglicht eine sequentielle Vermessung der Cornea mit einem einfachen Bilderfassungssystem.

In einer weiteren Ausführungsvariante werden die mehreren unterschiedlichen Reflexionsbilder aus dem selben von der Cornea auf Grund eines kontinuierlichen, zweidimensionalen Referenzmusters reflektierten Reflexionsmuster erzeugt und gespeichert. Das zweidimensionale Referenzmuster wird beispielsweise aus zwei zueinander phasenverschobenen, jeweils eine unterschiedliche Farbe aufweisenden periodischen Referenzgrundmustern erzeugt, und die den phasenverschobenen Referenzgrundmustern entsprechenden Reflexionsbilder werden durch Farbfiltrierung aus dem Reflexionsmuster erzeugt. Bei der Variante mit Referenzgrundmustern unterschiedlicher Farbe werden somit mehrere zueinander verschobene (z.B. kontinuierliche) zweidimensionale Referenzmuster mit unterschiedlicher Farbe gleichzeitig auf die Cornea projiziert respektive ausgestrahlt. In einer anderen Variante wird das zweidimensionale Referenzmuster mit Musterpunkten unterschiedlicher Polarisation erzeugt, und die Reflexionsbilder werden durch Polarisationsfiltrierung aus dem Reflexionsmuster erzeugt. Die Erzeugung eines Referenzmusters und die Erzeugung mehrerer darauf basierender Reflexionsbilder ermöglicht eine Vermessung der Cornea mit einem einzigen Bildprojektionsschritt, der keine Beeinflussung der Messgenauigkeit durch Augenbewegungen aufweist.

In einer weiteren Ausführungsvariante werden zwei zueinander um 180° phasenverschobene Referenzmuster erzeugt und es werden zwei Reflexionsbilder der von der Cornea auf Grund der zwei phasenverschobenen Referenzmuster reflektierten Reflexionsmuster gespeichert. Basierend auf den zwei Reflexionsbildern werden für die Cornea die Hintergrundintensität und die Signalmodulation berechnet. Die Hintergrundintensität wird beispielsweise basierend auf einer Addition der zwei Reflexionsbilder, und die Signalmodulation beispielsweise basierend auf einer Subtraktion der zwei Reflexionsbilder berechnet, wobei die addierten respektive subtrahierten Werte jeweils halbiert werden. Die separate Bestimmung von Hintergrundintensität und Signalmodulation ermöglicht eine nachfolgende Vermessung der Cornea, die mit bloss einem Bildprojektionsschritt auskommt, und somit keine oder nur eine geringfügige Beeinflussung der Messgenauigkeit durch Augenbewegungen aufweist.

Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

Figur 1: zeigt ein Blockdiagramm, das eine ophthalmologische Vorrichtung für die Vermessung einer Cornea illustriert, die schematisch im Querschnitt dargestellt ist.

Figur 2: zeigt ein Flussdiagramm, welches schematisch eine beispielhafte Folge von Schritten eines ophthalmologischen Verfahrens für die Vermessung einer Cornea illustriert.

Figuren 3 bis 6: zeigen Flussdiagramme, welche schematisch verschiedene Ausführungsvarianten mit unterschiedlichen Folgen von Schritten des ophthalmologischen Verfahrens für die Vermessung der Cornea illustrieren.

Figur 7a: zeigt schematisch eine Ansicht eines zweidimensionalen Referenzmusters.

Figur 7b: zeigt sägezahnförmige Modulationsverläufe für die Modulation eines physikalischen Lichtparameters des Referenzmusters.

Figur 7c: zeigt dreiecksförmige Modulationsverläufe für die Modulation eines physikalischen Lichtparameters des Referenzmusters.

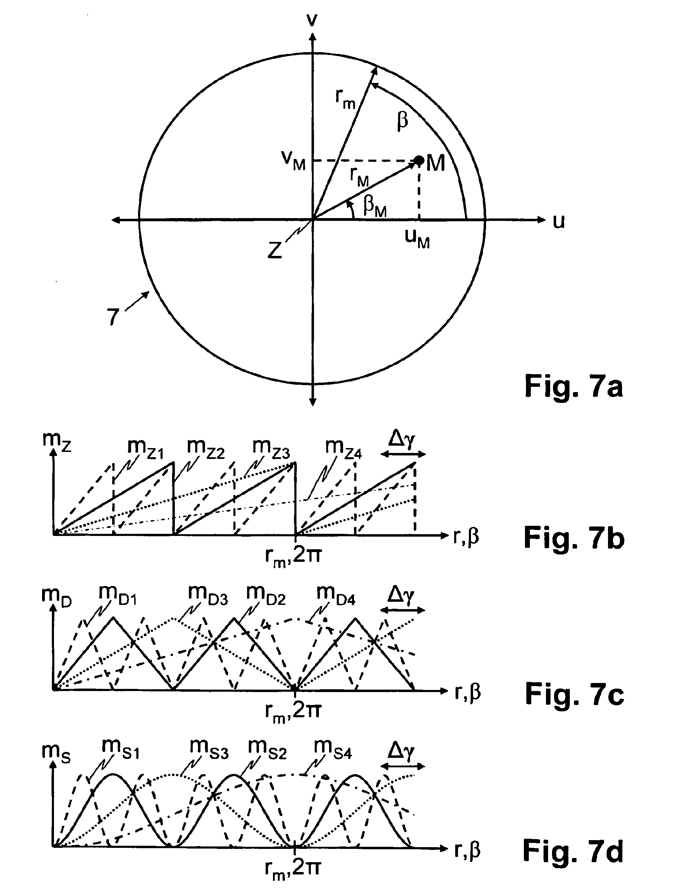

Figur 7c: zeigt sinusförmige Modulationsverläufe für die Modulation eines physikalischen Lichtparameters des Referenzmusters.

Wege zur Ausführung der Erfindung

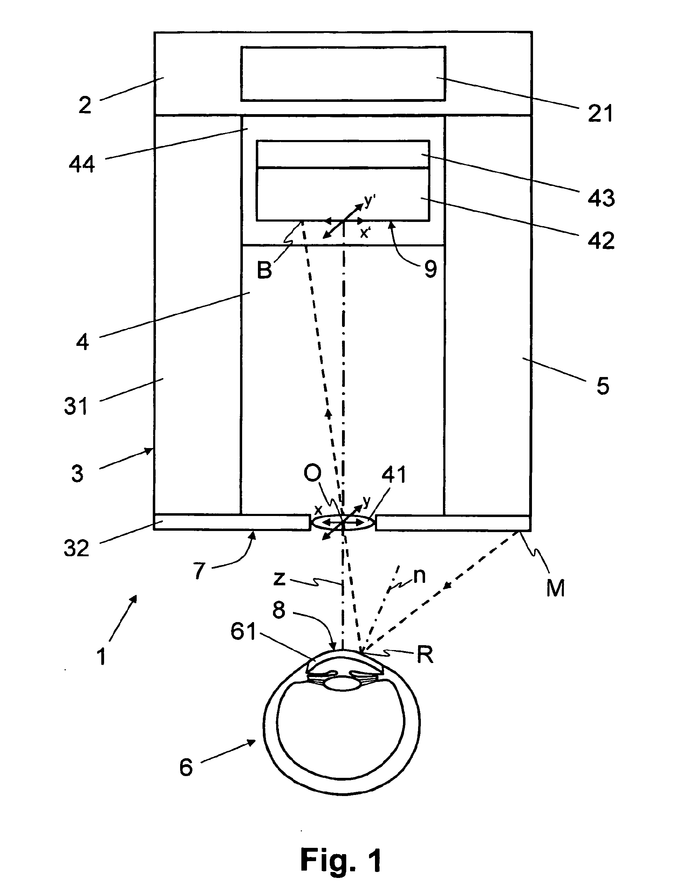

In der Figur 1 bezieht sich das Bezugszeichen 1 auf eine ophthalmologische Vorrichtung für die Vermessung einer Cornea 61, insbesondere eine Cornea eines menschlichen Auges 6.

Wie in der Figur 1 schematisch dargestellt ist, umfasst die ophthalmologische Vorrichtung 1 ein Verarbeitungsmodul 2, ein Referenzmustersystem 3, ein Bilderfassungssystem 4 und ein Ausgabemodul 5.

Das Verarbeitungsmodul 2 umfasst einen oder mehrere Computer mit einem oder mehreren Prozessoren, Daten- und Programmspeicher, sowie ein Steuermodul 21.

Das Referenzmustersystem 3 umfasst einen Referenzmustergenerator 31 zur Erzeugung von zweidimensionalen Referenzmustern 7 und einen Referenzmusterprojektor 32 zur Projektion und/oder Abstrahlung der erzeugten Referenzmuster 7 auf die Cornea 61. Der Referenzmusterprojektor 32 umfasst eine aktive Anzeige, beispielsweise eine LCD-Anzeige (Liquid Crystal Display) oder eine LED-Anzeige (Light Emitting Diode). In einer alternativen Ausführungsvariante umfasst der Referenzmusterprojektor 32 einen passiven Schirm und einen Projektor zur Projektion der Referenzmuster auf diesen Schirm. In einer Variante ist der aktive respektive passive Anzeigeschirm konkav ausgeführt, beispielsweise domförmig, trichterförmig, parabolisch oder ellipsoid konkav.

Das Bilderfassungssystem 4 umfasst eine optische Abbildungsvorrichtung 41, einen Bildwandler 42, einen Bildspeicher 43 und ein Extraktionsmodul 44. Der Bildwandler 42 umfasst beispielsweise einen CCD-Sensor (Charge Coupled Device). Die optische Abbildungsvorrichtung 41 umfasst eine oder mehrere optische Linsen. Die Abbildungsvorrichtung 41 ist eingerichtet ein von der Cornea 61 auf Grund eines projizierten/abgestrahlten Referenzmusters 7 spiegelnd reflektiertes Reflexionsmuster 8 auf den Bildwandler 42 abzubilden. Dabei wird ein Musterpunkt M des Referenzmusters 7 auf der Cornea 61 im Reflexionspunkt R des Reflexionsmusters 8 spiegelnd reflektiert und von der Abbildungsvorrichtung 41 im Bildwandler 42 fokussiert auf den Bildpunkt B des Reflexionsbilds 9 abgebildet. Da es sich um ein spiegelnde Abbildung handelt liegt nur ein virtuelles Bild vor, dass sich hinter der Cornea 61 befindet. Die im Bildwandler 42 erfassten Reflexionsbilder 9 der von der Cornea 61 reflektierten Reflexionsmuster 8 werden im Bildspeicher 43 zur weiteren Verarbeitung gespeichert.

Das Steuermodul 21, der Referenzmustergenerator 31 und das Extraktionsmodul 44 sind funktionale Module, die beispielsweise als programmierte Softwaremodule ausgeführt sind, welche Programmcode zur Steuerung eines Prozessors (des Verarbeitungsmoduls 2) umfassen, derart, dass der Prozessor die nachfolgend beschriebenen Funktionen ausführt. Der Fachmann wird verstehen, dass die funktionalen Module in einer Variante vollständig oder teilweise mit Hardwarekomponenten ausgeführt werden können. Insbesondere das Extraktionsmodul 44 kann optische Elemente zur optischen Ausführung von Bildverarbeitungsschritten umfassen, welche beispielsweise dem Bildwandler 42 vorgelagert oder in den Bildwandler 42 integriert sind. Die später detaillierter beschriebenen Funktionen des Extraktionsmoduls 44 können somit vollständig oder verteilt vor dem Bildwandler 42, im Bildwandler 42 und/oder basierend auf den gespeicherten Reflexionsbildern 9 (nach der Funktion des Bildwandlers 42) erfolgen.

Der Referenzmustergenerator 31 ist eingerichtet, die zweidimensionalen Referenzmuster 7 über den Referenzmusterprojektor 32 durch Modulation von einem oder mehreren physikalischen Lichtparameter zu erzeugen, insbesondere durch Modulation der Lichtintensität, der Lichtpolarisation und/oder der Lichtwellenlänge (Farbe). Wie in der Figur 7a dargestellt ist das Referenzmuster 7 beispielsweise kreisförmig und weist einen Radius $r_m$ auf. Jeder Musterpunkt M des Referenzmusters 7 ist durch seine kartesischen Koordinaten $(u_M, v_M)$ oder Polarkoordinaten $(r_M, \beta_M)$ definiert. Die Modulation der physikalischen Lichtparameter erfolgt im Referenzmuster 7 beispielsweise radial, d.h. abhängig vom Radius r und/oder abhängig vom (Kreis-) Winkel $\beta$. Der Fachmann wird verstehen, dass das Referenzmuster 7 in einer alternativen Ausführungsvariante auch entlang mehreren sich schneidenden Geraden oder Kurven moduliert werden kann, beispielsweise Spiderweb, Schachbrett oder hexagonale Muster. In einer weiteren Ausführungsvariante umfasst das Referenzmuster 7 neben dynamisch vom Referenzmustergenerator 31 erzeugten Referenzmustern 7 auch statische Grundmuster, die vom Referenzmustergenerator 31 den dynamisch erzeugten Referenzmustern 7 überlagert respektive unterlegt werden oder statisch auf dem Anzeigeschirm angebracht sind.

Die Figuren 7b-7d zeigen unterschiedliche Modulationsverläufe für die Modulation der physikalischen Lichtparameter des Referenzmusters 7. Figur 7b zeigt verschiedene sägezahnförmige Modulationsverläufe $m_Z$; Figur 7c zeigt verschiedene dreieckförmige Modulationsverläufe $m_D$; und Figur 7d zeigt verschiedene sinusförmige Modulationsverläufe $m_S$. Dabei weisen die Modulationsverläufe $m_{Z1}$, $m_{Z2}$, $m_{D1}$, $m_{D2}$, $m_{S1}$ und $m_{S2}$ zur Erzeugung periodischer Referenzmuster 7 jeweils eine Periode auf, die sich über den Radius $r=r_m$ respektive den Kreiswinkel $\beta=2\pi$ des Referenzmusters 7 mehrmals wiederholt. Die Modulationsverläufe $m_{Z3}$, $m_{D3}$ und $m_{S3}$ weisen jeweils eine Periode auf, die dem Radius $r=r_m$ respektive dem Kreiswinkel $\beta=2\pi$ des Referenzmusters 7 entspricht. Die Modulationsverläufe $m_{Z4}$, $m_{D4}$ und $m_{S4}$ weisen jeweils eine Periode auf, die grösser als der Radius $r=r_m$ respektive der Kreiswinkel $\beta=2\pi$ des Referenzmusters 7 ist.

In weiteren Ausführungsvarianten ist der Referenzmustergenerator 31 eingerichtet neben den erwähnten Modulationsverläufen $m_Z$, $m_D$ und $m_S$ auch andere durch Fourrierreihen beschreibbare Modulationsverläufe zu erzeugen. Der Referenzmustergenerator 31 ist zudem eingerichtet die verschiedenen Modulationsverläufe $m_Z$, $m_D$ und $m_S$, mit

einer Phasenverschiebung $\Delta\gamma$ zu erzeugen. Durch die Modulationsverläufe $m_Z$, $m_D$ und $m_S$ wird ein Referenzgrundmuster definiert, das durch die Phasenverschiebung $\Delta\gamma$ der Modulationsverläufe $m_Z$, $m_D$ und $m_S$ verschoben wird, somit können mehrere zueinander phasenverschobene Referenzmuster 7 durch Phasenverschiebung eines durch die Modulationsverläufe $m_Z$, $m_D$ und $m_S$ definierten periodischen Referenzgrundmusters erzeugt werden. Vorzugweise wird das Referenzmuster 7 so gestaltet, dass sich an allen Orten eine gleiche Phasenverschiebung $\Delta\gamma$ erzeugen lässt, wodurch sich die Verarbeitung vereinfacht, da im gesamten Bildbereich nur ein Auswerteverfahren angewendet werden muss. Eine Phasenverschiebung $\Delta\gamma$ im Punkt M des Referenzmusters 7 wirkt sich linear auf die Phasenverschiebung $\Delta\gamma'$ beim entsprechenden Bildpunkt B im Reflexionsbild 9 aus. In einer Ausführungsvariante ist der Referenzmustergenerator 31 überdies eingerichtet die Periode der verschiedenen Modulationsverläufe $m_Z$, $m_D$ und $m_S$ bei der Erzeugung der Referenzmuster 7 zu variieren, wobei sowohl die Intensität, also auch die Periode ortsabhängig sein können.

Das Ausgabemodul 5 umfasst eine oder mehrere Ausgabevorrichtungen, beispielsweise einen Bildschirm und/oder einen Drucker.

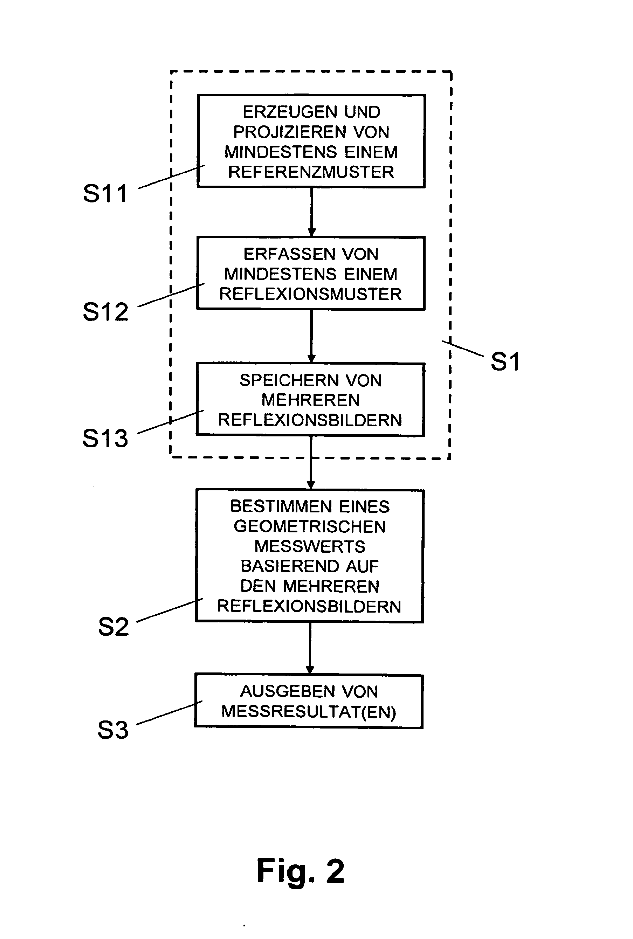

In den nachfolgenden Abschnitten werden das ophthalmologische Verfahren für die Vermessung der Cornea 61 sowie die Funktionen des Steuermoduls 21 und des Referenzmustergenerators 31 mit Bezug zu den Figuren 2-7 beschrieben.

Im Schritt S1 des ophthalmologischen Verfahrens werden zur Vermessung der Cornea 61 mehrere Reflexionsbilder 9 von einem oder mehreren von der Cornea 61 reflektierten Reflexionsmustern 8 bereitgestellt. Dazu werden vom Referenzmustersystem 3 im Schritt S11 eines oder mehrere unterschiedliche Referenzmuster 7 generiert und durch den Referenzmusterprojektor 32 auf die Cornea 61 projiziert respektive ausgestrahlt. Im Schritt S12, erfasst das Bilderfassungssystem 4 eines oder mehrere Reflexionsmuster 8, welche von der Cornea 61 auf Grund der im Schritt S11 erzeugten Referenzmuster 7 reflektiert werden. Im Schritt S13, werden im Bildspeicher 43 basierend auf dem einen oder den mehreren erfassten Reflexionsmustern 8 unterschiedliche Reflexionsbilder 9 gespeichert.

Im Schritt S2 bestimmt das Verarbeitungsmodul 2 mindestens einen geometrischen Messwert der Cornea 61 auf der Basis der im Schritt S13 gespeicherten Reflexionsbilder 9. Dazu berechnet das Steuermodul 21 für Punkte mit den Koordinaten x,y,z auf der Cornea 61, z.B. am Reflexionspunkt R des Reflexionsmusters 8, jeweils einen Phasenwert $\varphi$ des Reflexionsmusters 8 am betreffenden Punkt R, basierend auf den Intensitätswerten, die in den gespeicherten Reflexionsbildern 9 jeweils am dem betreffenden Punkt R entsprechenden Bildpunkt B ermittelt werden. Das Verarbeitungsmodul 2 berechnet den Phasenwert $\varphi$ des Reflexionsmusters 8 am betreffenden Punkt R mit den Koordinaten x, y, z beispielsweise durch Auswertung der untenstehenden Gleichung (1):

$$I(x', y') = I_0(x', y')\{1 + sm(x', y')\cos[\varphi(x', y')]\}, \qquad (1)$$

wobei $I(x', y')$ die gemessene Intensität am dem betreffenden Punkt R entsprechenden Bildpunkt B mit den Koordinaten $x', y'$, $I_0(x', y')$ die Hintergrundintensität am Bildpunkt B, $sm(x', y')$ eine Signalmodulation (z.B. Dämpfung) am Bildpunkt B, und $\varphi(x', y')$ der Phasenwert am Bildpunkt B ist. Dabei entspricht der Phasenwert $\varphi(x', y')$ am Bildpunkt B dem Phasenwert $\varphi(x, y)$ des Reflexionsmusters 8 am betreffenden Reflexionspunkt R auf der Cornea 61. Somit müssen mindestens drei unabhängige Informationen für die pixelgenaue Bestimmung des Phasenwerts $\varphi$ und der darauf basierenden geometrischen Messwerte bereitgestellt werden. Durch die Ermittlung der Intensität $I(x', y')$ für einen dem Reflexionspunkt R $(x,y,z)$ entsprechenden Bildpunkt B $(x', y')$ in mehreren gespeicherten Reflexionsbildern 9 können mehrere (z.B. drei) Gleichungen für die Berechnung der unbekannten Variablen Hintergrundintensität $I_0(x', y')$, Signalmodulation $sm(x', y')$ und Phasenwert $\varphi(x', y')$ am betreffenden Bildpunkt B aufgestellt, und darauf basierend der Phasenwert $\varphi(x', y')$ respektive $\varphi$ berechnet werden. Der Fachmann wird verstehen, dass für nicht-sinusförmige Modulationsverläufe, z.B. dreieckförmige Modulationsverläufe $m_D$ oder sägezahnförmige Modulationsverläufe $m_Z$, andere entsprechende Gleichungen zur Berechnung des gesuchten Phasenwerts $\varphi$ basierend auf den in den gespeicherten Reflexionsbildern 9 gemessenen Intensitäten $I(x', y')$ aufgestellt werden können. Bei der Vermessung der Cornea 61 über der Pupille des Auges 6 wird nur die Erzeugung von zwei Referenzmustern 7 respektive die Erfassung von zwei Reflexionsbildern 9 der von der Cornea 61 reflektierten Reflexionsmuster 8 benötigt, da bei der Pupille kein Hintergrundlicht vorliegt und die Signalmodulation konstant $sm(x', y')$ =1 1 ist.

Basierend auf den berechneten Phasenwerten bestimmt das Verarbeitungsmodul 2 einen oder mehrere (geometrische) Messwerte der Cornea 61, insbesondere die Oberflächenneigung, Oberflächenkrümmung, Oberflächenform (Topografie) und/oder Brechkraft der Cornea 61. Der Zusammenhang zwischen dem Bildpunkt B $(x', y')$ und dem Musterpunkt M $(u, v)$ respektive $(r, \beta)$ ergibt sich über die Messung des Phasenwerts $\varphi$ aus der Lage des Reflexionspunkts R $(x, y, z)$ auf der Cornea 61 und der Oberflächennormalen n an diesem Punkt (siehe in Figur 1 das durch die Punkte R, O, M definierte Dreieck). Unter Annahmen bzw. zusätzlicher Messungen des Abstands z des Reflexionspunkts R bezüglich des im Nullpunkt O angesetzten Koordinatensystems $(x,y,z)$ der Messvorrichtung 1, kann auf die Koordinaten $x,y$ geschlossen werden. Mit der der Lage des Reflexionspunkts R $(x,y,z)$, kann die Oberflächenneigung bestimmt werden. Über Integration der Oberflächenneigungen an einer Vielzahl von Reflexionspunkten R (*x,y,z*) ergibt sich die Oberflächenform. Je mehr Abstandswerte respektive Reflexionspunkte R (*x,y,z*) bekannt sind (oft zumindest der Apex) und je dichter die Messpunkte der Oberflächenneigung liegen und je genauer die Oberflächenneigung gemessen werden kann, desto genauer kann die Oberflächenform bestimmt werden. Oberflächenkrümmungen lassen sich entweder über Ableitungen der Oberflächenneigung oder über eingepasste Sphären definieren.

Im Schritt S3 gibt das Steuermodul 21 die im Schritt S2 bestimmten Messwerte über das Ausgabemodul 5 an einen Benutzer aus. Das Ausgabemodul 5 erzeugt eine Darstellung der ermittelten Messwerte wie Oberflächenneigung, Oberflächenkrümmung, Oberflächenform (Topografie) und/oder Brechkraft der Cornea 61 grafisch und/oder numerisch auf einer Anzeige oder als Ausdruck.

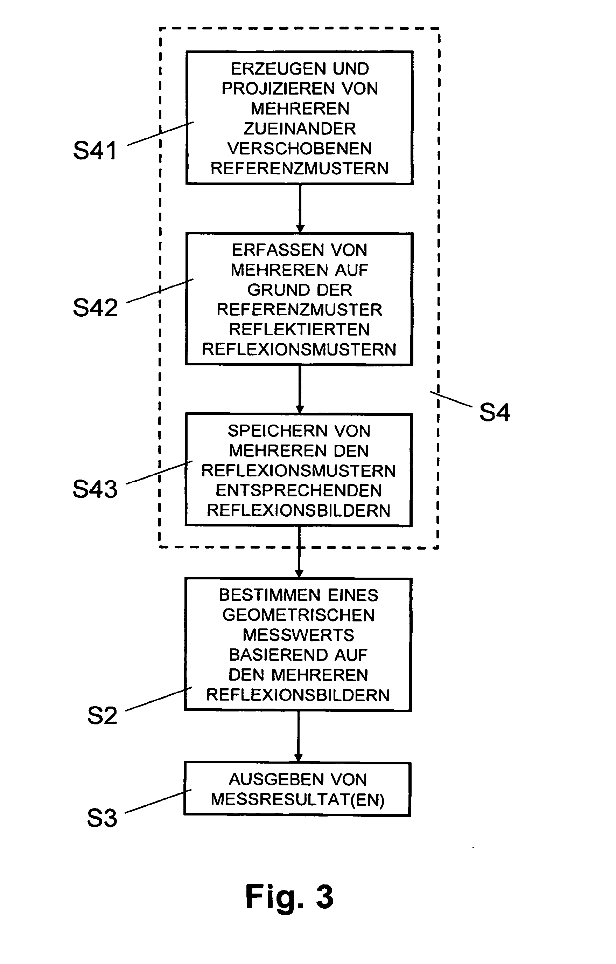

In der Ausführungsvariante gemäss Figur 3 wird der oben mit Bezug zur Figur 2 beschriebene Schritt S1 des ophthalmologischen Verfahrens durch Ausführung des entsprechenden Schritts S4 bewirkt.

Im Schritt S41 werden vom Referenzmustersystem 3 mehrere zueinander verschobene Referenzmuster 7 generiert und durch den Referenzmusterprojektor 32 auf die Cornea 61 projiziert respektive ausgestrahlt. Dazu aktiviert das Steuermodul 21 den Referenzmustergenerator 31, welcher mehrere, beispielsweise drei, zueinander phasenverschobene Referenzmustern 7 erzeugt und auf die Cornea 61 projiziert respektive ausstrahlt.

Im Schritt S42 erfasst das Bilderfassungssystem 4 die Sequenz der Reflexionsmuster 8, die von der Cornea 61 auf Grund der im Schritt S41 erzeugten Sequenz von Referenzmustern 7 reflektiert werden.

Im Schritt S43 werden die Reflexionsbilder 9 der von der Cornea 61 reflektierten Reflexionsmuster 8 im Bildspeicher 43 einander zugeordnet gespeichert. Der Fachmann wird verstehen, dass die Schrittfolge schematisch dargestellt ist und dass sich die Folge der Schritte S41, S42, S43 mindestens dreimal wiederholt, jeweils einmal für die Erzeugung eines Referenzmusters 7 und die Erfassung des darauf basierenden Reflexionsmusters 8 respektive Reflexionsbilds 9.

Im nachfolgenden Schritt S2 bestimmt das Verarbeitungsmodul 2 wie oben mit Bezug zur Figur 2 beschrieben mindestens einen geometrischen Messwert der Cornea 61 basierend auf den gespeicherten Reflexionsbildern 9 der Reflexionsmuster 8. Im Schritt S3 werden die bestimmten Messwerte wie oben mit Bezug zur Figur 2 beschrieben über das Ausgabemodul 5 an den Benutzer ausgegeben.

An dieser Stelle soll zudem angeführt werden, dass sich bei der Erzeugung der drei zueinander phasenverschobenen Referenzmuster 7 im Schritt S41 für bestimmte Werte der Phasenverschiebung $\Delta\gamma$ besonders einfache Gleichungen für die mit Bezug zur Figur 2 beschriebene Berechnung des Phasenwerts $\varphi$ basierend auf den in den gespeicherten Reflexionsbildern 9 gemessenen Intensitäten $I_1(x',y')$, $I_2(x',y')$ und $I_3(x',y')$ ergeben.

Beispielsweise ergibt sich für die Phasenverschiebung von $\Delta\gamma=\Pi/2$ zwischen den Referenzmustern 7 (d.h. $\Delta\gamma_1=0$, $\Delta\gamma_2=\Pi/2$, $\Delta\gamma_3=\Pi$) die folgende einfache Gleichung:

$$\varphi(x',y') = \arctan\left(\frac{I_1(x',y') - 2I_2(x',y') + I_3(x',y')}{I_1(x',y') - I_3(x',y')}\right) (2)$$

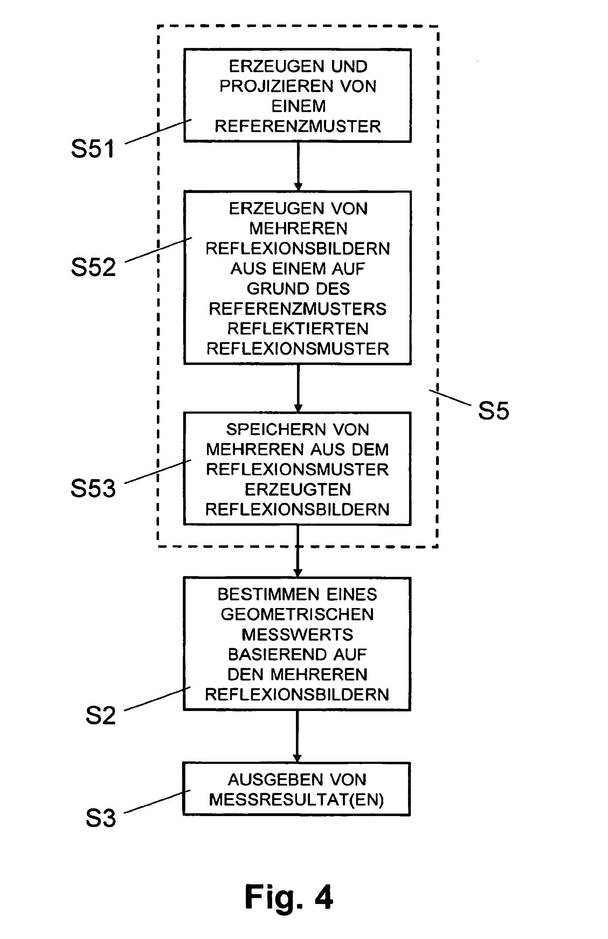

In der Ausführungsvariante gemäss Figur 4 wird der oben mit Bezug zur Figur 2 beschriebene Schritt S1 des ophthalmologischen Verfahrens durch Ausführung des entsprechenden Schritts S5 bewirkt.

Im Schritt S51 wird vom Referenzmustersystem 3 ein Referenzmuster 7 generiert und durch den Referenzmusterprojektor 32 auf die Cornea 61 projiziert respektive ausgestrahlt. Dazu aktiviert das Steuermodul 21 den Referenzmustergenerator 31, welcher das Referenzmuster 7 je nach Ausführungsvariante mit verschiedenfarbigen oder unterschiedlich polarisierten Referenzgrundmustern generiert, d.h. das Referenzmuster 7 wird aus mindestens zwei zueinander phasenverschobenen, jeweils eine unterschiedliche Farbe oder Musterpunkte M unterschiedlicher Polarisation aufweisenden (periodischen) Referenzgrundmustern erzeugt. Das Referenzmuster 7 wird beispielsweise aus zwei zueinander um $\Delta\gamma=\Pi/2$ (90°) phasenverschobenen Referenzgrundmustern mit unterschiedlichen Farben (Farbkanäle) erzeugt. Im Schritt S51 werden somit mehrere zueinander verschobene (z.B. kontinuierliche) zweidimensionale Referenzmuster respektive Referenzgrundmuster mit unterschiedlicher Farbe (z.B. drei verschiedene Farben) respektive unterschiedlicher Polarisation gleichzeitig auf die Cornea 61 projiziert respektive ausgestrahlt.

Im Schritt S52 erfasst das Bilderfassungssystem 4 das Reflexionsmuster 8, das von der Cornea 61 auf Grund des im Schritt S51 erzeugten Referenzmusters 7 reflektiert wird, und speichert das Reflexionsmuster 8 im Bildspeicher 43. Im Schritt S52 werden somit die mehreren im Schritt S51 gleichzeitig projizierten respektive ausgestrahlten Referenzmuster respektive Referenzgrundmuster erfasst. Das Extraktionsmodul 44 ist eingerichtet, im Schritt S52 aus dem gespeicherten Reflexionsmuster 8 mehrere (mindestens zwei) Reflexionsbilder 9 zu erzeugen und im Bildspeicher 43 einander zugeordnet zu speichern. Wie bereits oben erwähnt wurde, ist das Extraktionsmodul 44 in alternativen Ausführungsvarianten dem Bildwandler 42 vorgelagert oder in den Bildwandler 42 integriert, so dass die Reflexionsbilder 9 nicht erst basierend auf dem gespeicherten Reflexionsmuster 8 erzeugt werden, sondern jeweils ohne Zwischenspeicherung des Reflexionsmusters 8 direkt optisch aus dem auf der Cornea 61 reflektierten Reflexionsmuster 8 erzeugt werden. Abhängig von der betreffenden Ausführungsvariante umfasst das Extraktionsmodul 44 Farbfilter zur Erzeugung der Reflexionsbilder 9 durch Farbfiltrierung aus dem Reflexionsmuster 8, oder Polarisationsfilter zur Erzeugung der Reflexionsbilder 9 durch Polarisationsfiltrierung aus dem Reflexionsmuster 8. Zur Erzeugung der Reflexionsbilder 9 durch Polarisationsfiltrierung umfasst das Bilderfassungssystem 4 in einer Ausführungsvariante mehrere (z.B. zwei oder drei) koaxiale optische Kanäle, z.B. mehrere (CCD) Kameras, die jeweils das gleiche Reflexionsmuster 8 durch unterschiedlich orientierte Polarisationsfilter erfassen. Dadurch wird eine Polarisationsmessung basierend auf mehreren Bildern unterschiedlicher Polarisation ermöglicht. Entsprechend lassen sich die Polarisationsfilter durch Farbfilter und Farbfiltrierung ersetzen. Auch eine Kombination von Farb- und Polarisationsfiltrierung ist möglich.

Im Schritt S53 werden die im Schritt S52 aus dem Reflexionsmuster 8 erzeugten Reflexionsbilder 9 im Bildspeicher 43 einander zugeordnet gespeichert.

Im nachfolgenden Schritt S2 bestimmt das Verarbeitungsmodul 2 basierend auf den erzeugten Reflexionsbildern 9, wie oben mit Bezug zur Figur 2 beschrieben, einen oder mehrere geometrische Messwerte der Cornea 61, die im Schritt S3 über das Ausgabemodul 5 an den Benutzer ausgegeben werden.

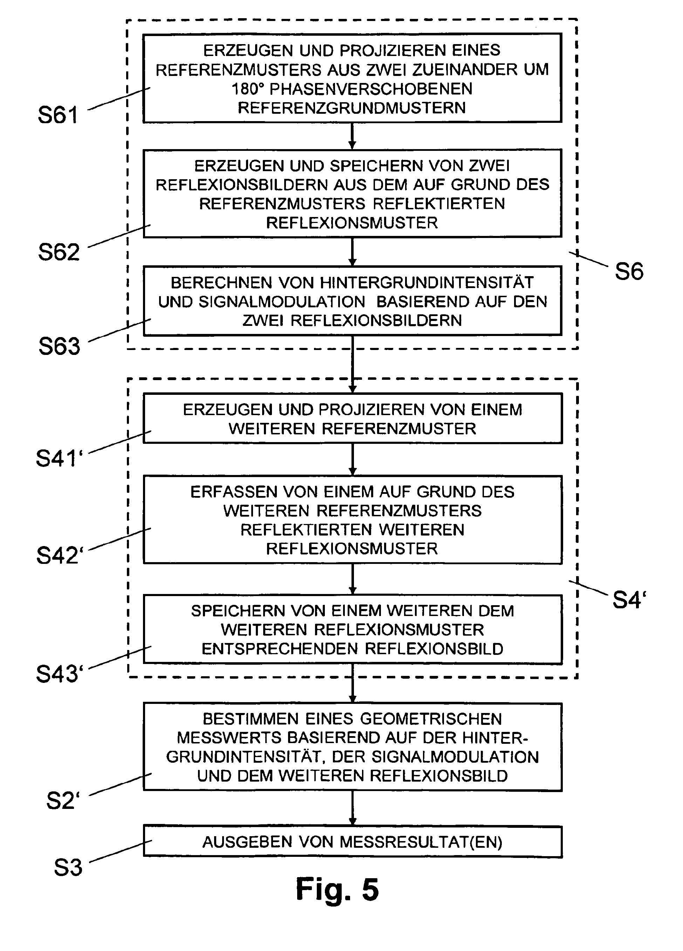

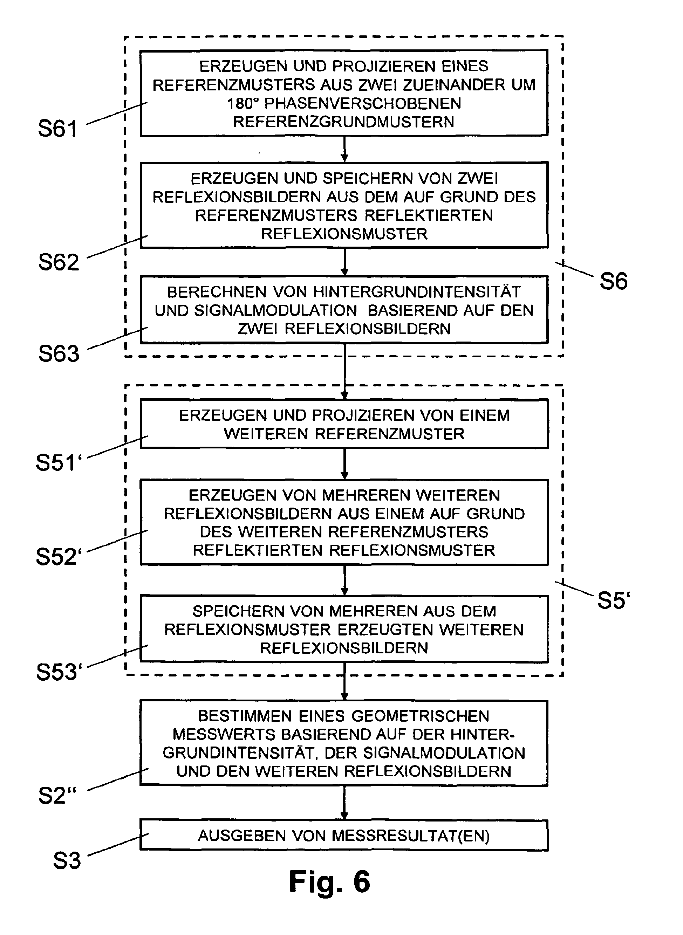

Die Ausführungsvarianten gemäss den Figuren 5 und 6 weisen jeweils einen Schritt S6 zur separaten Bestimmung der Hintergrundintensität $I_0(x,y')$ an der Cornea 61 und der auf der Übertragung zur Cornea 61 bewirkten Signalmodulation $sm(x',y')$ auf.

Im Schritt S61 wird vom Referenzmustersystem 3 ein Referenzmuster 7 aus zwei zueinander um die Phasenverschiebung $\Delta\gamma = \Pi$ (180°) verschobenen bzw. invertierten Referenzgrundmustern generiert und durch den Referenzmusterprojektor 32 auf die Cornea 61 projiziert respektive ausgestrahlt. Dazu aktiviert das Steuermodul 21, wie oben mit Bezug zu Schritt S51 beschrieben, den Referenzmustergenerator 31, welcher das kombinierte Referenzmuster 7 je nach Ausführungsvariante aus zwei zueinander um $\Delta\gamma = \Pi$ phasenverschobenen, jeweils eine unterschiedliche Farbe oder Musterpunkte M unterschiedlicher Polarisation aufweisenden (periodischen) Referenzgrundmustern erzeugt.

Im Schritt S62, erzeugt das Extraktionsmodul 44, wie oben mit Bezug zu Schritt S52 beschrieben, basierend auf dem Reflexionsmuster 8, das von der Cornea 61 auf Grund des im Schritt S61 erzeugten Referenzmusters 7 reflektiert wird, zwei Reflexionsbilder 9 und speichert diese im Bildspeicher 43 einander zugeordnet ab.

Im Schritt S63 berechnet das Steuermodul 21 basierend auf den beiden im Schritt S62 erfassten Reflexionsbildern 9 die Hintergrundintensität $I_0(x',y')$ und die Signalmodulation $sm(x',y')$. Dabei wird die Hintergrundintensität $I_0(x',y')$ durch Addition der beiden Reflexionsbilder 9 und Division durch den Faktor zwei berechnet, d.h. bei einer Repräsentation der Reflexionsbilder 9 als Matrix von Bildpunkten mit einem Intensitätswert $I(x', y')$, werden die Intensitätswerte $I(x', y')$ von Matrixelementen der Reflexionsbilder 9 mit den selben Indizes $x',y'$ addiert und durch Zwei dividiert. Die Signalmodulation $sm(x', y')$ wird durch Subtraktion der beiden Reflexionsbilder 9 und Division durch den Faktor zwei berechnet, d.h. die Intensitätswerte $I(x', y')$ der Matrixelemente mit den selben Indizes $x',y'$ werden subtrahiert und durch Zwei dividiert, und gegebenenfalls mit einem weiteren Faktor für die nachfolgende Verarbeitung gewichtet. Bei binären Referenzmustern 7 liegt die berechnete Signalmodulation $sm(x',y')$ als numerischer Wert vor. Bei kontinuierlichen Referenzmustern 7 ergibt sich durch die Subtraktion zweier Intensitätswerte $I_1(x',y')$ und $I_2(x',y')$ eines betreffenden Punkts $(x',y')$ für die Signalmodulation $sm(x', y')$ für den Fall sinusförmiger Modulationsverläufe der untenstehende Ausdruck:

$$I_1(x',y') - I_2(x',y') = 2I_0(x',y')sm(x',y')\cos(\varphi(x',y')), (3)$$

wobei die die Hintergrundintensität $I_0(x',y')$ aus der Addition der beiden Reflexionsbilder 9 bekannt ist und der Phasenwert $\varphi$ in einem nachfolgenden Schritt berechnet werden muss, wie nachfolgend mit Bezug zu den Figuren 5 und 6 beschrieben wird.

Gemäss der Ausführungsvariante nach Figur 5, wird im Schritt S4' zur Auswertung der Gleichung (1) ein weiteres Referenzmuster 7 hinzugezogen. Im Schritt S41' wird vom Referenzmustersystem 3 respektive vom Referenzmustergenerator 31 ein weiteres Referenzmuster 7 generiert und durch den Referenzmusterprojektor 32 auf die Cornea 61 projiziert respektive ausgestrahlt. Vorzugsweise weist das im Schritt S41' erzeugte weitere Referenzmuster 7 eine mittlere Intensität auf, die der mittleren Intensität der zwei im Schritt S61 generierten zueinander um $\Delta\gamma = \pi$ (180°) verschobenen Referenzmuster 7 entspricht. Das weitere Referenzmuster 7 weist in einer Variante zu den beiden im Schritt S61 generierten Referenzmustern beispielsweise eine Phasenverschiebung von $\Delta\gamma = \pi/2$ (90°) auf.

Im Schritt S42' erfasst das Bilderfassungssystem 4 das weitere Reflexionsmuster 8, das von der Cornea 61 auf Grund des weiteren Referenzmusters 7 reflektiert wird.

Im Schritt S43' wird das Reflexionsbild 9 des von der Cornea 61 reflektierten weiteren Reflexionsmusters 8 im Bildspeicher 43 den im Schritt S6 berechneten Hintergrundintensität $I_0(x',y')$ und Signalmodulation $sm(x',y')$ zugeordnet gespeichert.

Im Schritt S2' bestimmt das Verarbeitungsmodul 2 respektive das Steuermodul 21 für Punkte R mit den Koordinaten (x, y, z) auf der Cornea 61 jeweils einen Phasenwert φ des weiteren Reflexionsmusters 8 gemäss Gleichung (1) im Falle von sinusförmigen Referenzmustern 7 (d.h. Referenzmuster 7 mit sinusförmigen Modulationsverläufen), basierend auf den im Schritt S6 berechneten Hintergrundintensität $I_0(x',y')$ und Signalmodulation $sm(x',y')$, sowie basierend auf dem Intensitätswert $I(x',y')$, der im gespeicherten Reflexionsbild 9 am entsprechenden Bildpunkt B ermittelt wird, wobei je nach Ausführungsvariante die Phasenverschiebung $\Delta\gamma$ des weiteren Referenzmusters 7 mitberücksichtigt wird. Bei der Verwendung von binären Referenzmustern 7 im Schritt S6 wird zur Berechnung des Phasenwerts φ der numerische Wert der berechneten Signalmodulation $sm(x', y')$ in die Gleichung (1) eingesetzt. Bei der Verwendung von kontinuierlichen sinusförmigen Referenzmustern 7 im Schritt S6 wird der Phasenwert φ durch Auflösung der beiden Gleichungen (1) und (3) berechnet. Basierend auf dem berechneten Phasenwert φ bestimmt das Verarbeitungsmodul 2 einen oder mehrere (geometrische) Messwerte der Cornea 61 wie oben mit Bezug zur Figur 2 beschrieben wurde. Bei andersförmigen Referenzmustern müssen analoge Gleichungen verwendet werden.

Im Schritt S3 werden die bestimmten Messwerte wie oben mit Bezug zur Figur 2 beschrieben über das Ausgabemodul 5 an den Benutzer ausgegeben.

Gemäss der Ausführungsvariante nach Figur 6, wird im Schritt S5' zur Auswertung der Gleichung (1) ein weiteres Referenzmuster 7 hinzugezogen.

Im Schritt S51' wird vom Referenzmustersystem 3 respektive vom Referenzmustergenerator 31 ein weiteres Referenzmuster 7 generiert und durch den Referenzmusterprojektor 32 auf die Cornea 61 projiziert respektive ausgestrahlt. Vorzugsweise weist das im Schritt S51' erzeugte weitere Referenzmuster 7 eine mittlere Intensität auf, die der mittleren Intensität der zwei im Schritt S61 generierten zueinander um $\Delta\gamma = \pi$ (180°) verschobenen Referenzmuster 7 entspricht. Das weitere Referenzmuster 7 wird je nach Ausführungsvariante mit verschiedenfarbigen und/oder unterschiedlich polarisierten Referenzgrundmustern generiert, wie oben mit Bezug zur Figur 4 beschrieben wurde.

In der Ausführungsvariante mit verschiedenfarbigen, z.B. um $\Delta\gamma = \pi/2$ (90°) phasenverschobenen, Referenzgrundmustern werden die im Schritt S6 bestimmte Hintergrundintensität $I_0(x',y')$ und Signalmodulation $sm(x', y')$ jeweils für beide verwendeten Farbkanäle (d.h. für die Referenzgrundmuster beider verwendeten Farben) bestimmt.

Im Schritt S52' erfasst das Bilderfassungssystem 4 das Reflexionsmuster 8, das von der Cornea 61 auf Grund des im Schritt S51' erzeugten Referenzmusters 7 reflektiert wird, und das Extraktionsmodul 44 erzeugt aus dem erfassten Reflexionsmuster 8, wie oben mit Bezug zur Figur 4 beschrieben wurde, durch Farbfiltrierung respektive Polarisationsfiltrierung aus dem Reflexionsmuster 8 mehrere weitere Reflexionsbilder 9.

Im Schritt S53' werden die erzeugten weiteren Reflexionsbilder 9 im Bildspeicher 43 den im Schritt S6 berechneten Hintergrundintensität $I_0(x',y')$ und Signalmodulation $sm(x',y')$ zugeordnet gespeichert, wobei evtl. Augenbewegungen bei der zugeordneten Speicherung berücksichtig werden.

Im Schritt S2" bestimmt das Steuermodul 21 für Punkte R mit den Koordinaten (x, y, z) auf der Cornea 61 jeweils den Phasenwert φ des Reflexionsmusters 8, basierend auf der im Schritt S6 berechneten Hintergrundintensität $I_0(x',y')$ und Signalmodulation $sm(x',y')$ sowie den Intensitätswerten $I(x',y')$, die in den gespeicherten Reflexionsbildern 9 am entsprechenden Bildpunkt ermittelt werden. Bei der Verwendung von binären Referenzmustern 7 im Schritt S6 wird zur Berechnung des Phasenwerts φ der numerische Wert der berechneten Signalmodulation $sm(x',y')$ in die Gleichung (1) eingesetzt. Bei der Verwendung von kontinuierlichen sinusförmigen Referenzmustern 7 im Schritt S6 wird der Phasenwert φ durch Auflösung der beiden Gleichungen (1) und (3) berechnet. Da die Gleichung (1) mit den mehreren gespeicherten Reflexionsbildern 9 überbestimmt ist, wird für die Reflexionsbilder 9 jeweils ein separater Phasenwert $\varphi_i(x',y')$ bestimmt und durch Mittelwertbildung daraus ein resultierender Phasenwert $\varphi(x',y')$ berechnet, welcher Messungenauigkeiten in der Bestimmung der separaten Phasenwerte $\varphi_i(x',y')$ ausmittelt. Basierend auf dem berechneten Phasenwert $\varphi(x',y')$ bestimmt das Verarbeitungsmodul 2 einen oder mehrere (geometrische) Messwerte der Cornea 61, und im Schritt S3 werden die bestimmten Messwerte, wie oben mit Bezug zur Figur 2 beschrieben, über das Ausgabemodul 5 an den Benutzer ausgegeben.

Ein Vorteil des in den Figuren 5 und 6 dargestellten Verfahrens ist die reduzierte Anzahl gleichzeitig zu erfassender Bilder. Im Falle des Einsatzes von drei CCD Kameras können somit eine Kamera und die dazugehörige optischen Elemente eingespart werden.

Abschliessend soll angeführt werden, dass die ophthalmologische Vorrichtung 1 in weiteren Ausführungsvarianten zur Verbesserung der Messgenauigkeit Fixiertargets, Augenabstandsmessvorrichtungen, Eyetrackers und/oder Mittel zur Erfassung und Auswertung überbestimmter Messdaten, d.h. mehr als die mindestens erforderliche Anzahl Referenzbilder 9, umfasst.

Es soll nochmals explizit festgehalten werden, dass die Verwendung von kontinuierlichen Mustern zwar die die Messpunktzahl erhöht, dass aber erst durch die Verwendung mehrerer Bilder sich die Messgenauigkeit erhöht aufgrund der Elimination der Hintergrundbeleuchtung und der Berücksichtigung der Signalmodulation. Weiterhin ermöglicht die Verwendung mehrerer Bilder die zusätzliche Berechnung von Gütewerten zur Beurteilung der Messgenauigkeit. Die zusätzliche Berechung der Signalmodulation ergibt Aufschluss über Signal-zu-Rausch-Verhältnisse und damit die Messunsicherheit. Eine Berechnung des Phasenschiebewinkels lässt auf Augenbewegungen schliessen. Eine Anpassung des Referenzmusters (z.B. im Falle von zu dichten Streifen aufgrund zu dichter Perioden im reflektierten Reflexionsmuster) an das zu messende Auge kann mit Hilfe der Güteparameter vorgenommen werden.

Schliesslich soll hier angeführt werden, dass in der Beschreibung zwar Computerprogrammcode spezifischen funktionalen Modulen zugeordnet wurde und dass die Ausführung von Schritten in einer bestimmten Reihenfolge dargestellt wurde, dass der Fachmann jedoch verstehen wird, dass der Computerprogrammcode unterschiedlich strukturiert und die Reihenfolge von mindestens gewissen Schritten geändert werden kann, ohne dabei vom (durch die Ansprüche definierten) Schutzgegenstand abzuweichen.

**Patentansprüche**

1. Ein ophthalmologisches Verfahren zur Vermessung einer Cornea (61), umfassend:

    Erzeugen eines zweidimensionalen Referenzmusters (7) durch einen Referenzmustergenerator (31) aus mindestens zwei durch Phasenverschiebung zueinander verschobenen, jeweils eine unterschiedliche Farbe oder Musterpunkte (M) unterschiedlicher Polarisation aufweisenden periodischen Referenzgrundmustern;
    Projektion (S11) des erzeugten zweidimensionalen Referenzmusters (7) auf die Cornea (61); und
    Erfassen (S12) eines von der Cornea (61) auf Grund des Referenzmusters (7) reflektierten Reflexionsmusters (8);
    Erzeugen von mehreren unterschiedlichen Reflexionsbildern (9) aus dem von der Cornea (61) auf Grund des zweidimensionalen Referenzmusters (7) reflektierten Reflexionsmuster (8) durch Farbfiltrierung oder Polarisationsfiltrierung;
    Speichern (S13) der mehreren unterschiedlichen Reflexionsbilder (9); **gekennzeichnet durch** Berechnen für Punkte (R) auf der Cornea (61) jeweils eines Phasenwerts ($\varphi$) des Reflexionsmusters (8) basierend auf in den gespeicherten Reflexionsbildern (9) jeweils an einem dem betreffenden Punkt (R) entsprechenden Bildpunkt (B) gemessenen Intensitäten ; und
    Bestimmen (S2) mindestens eines geometrischen Messwerts der Cornea (61) basierend auf den Phasenwerten ($\varphi$).

2. Verfahren nach Anspruch 1, **dadurch gekennzeichnet, dass** mehrere, durch Phasenverschiebung zueinander verschobene kontinuierliche, zweidimensionale Referenzmuster (7) zur jeweiligen Projektion auf die Cornea (61) erzeugt (S41) werden, und dass jeweils ein Reflexionsbild (9) von von der Cornea (61) auf Grund der verschobenen Referenzmuster (7) reflektierten Reflexionsmustern (8) gespeichert (S43) wird.

3. Verfahren nach Anspruch 2, **dadurch gekennzeichnet, dass** jeweils ein Reflexionsbild (9) von von der Cornea (61) auf Grund der phasenverschobenen Referenzmuster (7) reflektierten Reflexionsmustern (8) gespeichert (S43) wird.

4. Verfahren nach Anspruch 1, **dadurch gekennzeichnet, dass** die mehreren unterschiedlichen Reflexionsbilder (9) aus dem selben von der Cornea (61) auf Grund eines kontinuierlichen, zweidimensionalen Referenzmusters (7) reflektierten Reflexionsmuster (8) erzeugt (S52) und gespeichert (S53) werden.

5. Verfahren nach Anspruch 4, **dadurch gekennzeichnet, dass** das zweidimensionale Referenzmuster (7) aus zwei zueinander phasenverschobenen, jeweils eine unterschiedliche Farbe aufweisenden periodischen Referenzgrundmustern erzeugt (S51) wird, und dass die den phasenverschobenen Referenzgrundmustern (7) entsprechenden Reflexionsbilder (9) durch Farbfiltrierung aus dem Reflexionsmuster (8) erzeugt (S52) werden.

6. Verfahren nach Anspruch 4, **dadurch gekennzeichnet, dass** das zweidimensionale Referenzmuster (7) mit Musterpunkten (M) unterschiedlicher Polarisation erzeugt (S51) wird, und dass die Reflexionsbilder (9) durch Polarisationsfiltrierung aus dem Reflexionsmuster erzeugt (S52) werden.

7. Verfahren nach einem der Ansprüche 1 bis 6, **dadurch gekennzeichnet, dass** für Punkte (R) auf der Cornea (61) basierend auf den Reflexionsbildern (9) jeweils der Phasenwert ($\varphi$) des Reflexionsmusters (8) am betreffenden Punkt (R) durch Auswertung der Gleichung

$$I(x', y') = I_0(x', y')\{1 + m(x', y')\cos[\varphi(x', y')]\},$$

berechnet wird, wobei $I(x', y')$ eine gemessene Intensität am entsprechenden Bildpunkt (B) mit Koordinaten $x',y'$, $I_0(x',y')$ eine Hintergrundintensität am Bildpunkt (B), $m(x', y')$ eine Signalmodulation am Bildpunkt (B), und $\varphi(x', y')$ der Phasenwert am betreffenden Punkt (R) ist.

8. Verfahren nach Anspruch 7, **dadurch gekennzeichnet, dass** zwei zueinander um 180° phasenverschobene Referenzmuster (7) erzeugt (S61) werden; dass zwei Reflexionsbilder (9) der von der Cornea (61) auf Grund der zwei phasenverschobenen Referenzmuster (7) reflektierten Reflexionsmuster (8) gespeichert (S62) werden; und dass basierend auf den zwei Reflexionsbildern (9) für die Cornea (61) die Hintergrundintensität und die Signalmodulation berechnet (S63) werden.

9. Verfahren nach Anspruch 8, **dadurch gekennzeichnet, dass** die Hintergrundintensität basierend auf einer Addition der zwei Reflexionsbilder (9), und die Signalmodulation basierend auf einer Subtraktion der zwei Reflexionsbilder (9) berechnet werden.

10. Verfahren nach einem der Ansprüche 1 bis 9, **dadurch gekennzeichnet, dass** die Referenzmuster (7) durch Modulation von mindestens einem physikalischen Lichtparameter aus Lichtintensität, Lichtpolarisation und Lichtwellenlänge erzeugt werden.

11. Verfahren nach einem der Ansprüche 1 bis 10 **dadurch gekennzeichnet, dass** ein periodisches Referenzmuster (7) mit einer radial verlaufenden und beispielsweise variierbaren Periode erzeugt wird.

12. Eine ophthalmologische Vorrichtung (1) zur Vermessung einer Cornea (61), umfassend:

einen Referenzmustergenerator (31) eingerichtet zum Erzeugen eines zweidimensionalen Referenzmusters (7) aus mindestens zwei durch Phasenverschiebung zueinander verschobenen, jeweils eine unterschiedliche Farbe oder Musterpunkte (M) unterschiedlicher Polarisation aufweisenden periodischen Referenzgrundmustern;
ein Referenzmustersystem (3) eingerichtet zur Projektion des erzeugten zweidimensionalen Referenzmusters (7) auf die Cornea (61);
ein Bilderfassungssystem (4) eingerichtet zur Erfassung eines von der Cornea (61) auf Grund des Referenzmusters (7) reflektierten Reflexionsmusters (8);
ein Extraktionsmodul (44) eingerichtet zum Erzeugen von mehreren unterschiedlichen Reflexionsbildern (9) aus dem von der Cornea (61) auf Grund des zweidimensionalen Referenzmusters (7) reflektierten Reflexionsmuster (8) durch Farbfiltrierung oder Polarisationsfiltrierung;
wobei das Bilderfassungssystem (4) eingerichtet ist, die mehreren unterschiedlichen Reflexionsbilder (9) zu speichern; **gekennzeichnet durch** ein Verarbeitungsmodul (2), welches eingerichtet ist, für Punkte (R) auf der Cornea (61) jeweils ein Phasenwert ($\varphi$) des Reflexionsmusters (8) basierend auf in den gespeicherten Reflexionsbildern (9) jeweils an einem dem betreffenden Punkt (R) entsprechenden Bildpunkt (B) gemessenen Intensitäten zu berechnen, und mindestens einen geometrischen Messwert der Cornea (61) basierend auf dem Phasenwert ($\varphi$) zu bestimmen.

13. Vorrichtung (1) nach Anspruch 12, **dadurch gekennzeichnet, dass** das Referenzmustersystem (3) eine aktive Anzeige, insbesondere eine LCD-Anzeige, oder eine Kombination eines passiven Schirms mit einem Projektor zur Projektion der Referenzmuster (7) auf den Schirm umfasst.

## Claims

1. Ophthalmological method for measuring a cornea (61), comprising:

generation of a two-dimensional reference pattern (7) by a reference pattern generator (31) from at least two mutually phase-shifted periodic basic reference patterns respectively having a different colour or pattern points (M) of different polarization;

projection (S11) of the generated two-dimensional reference pattern (7) onto the cornea (61); and acquisition (S12) of a reflection pattern (8) reflected by the cornea (61) by virtue of the reference pattern (7); generation of a plurality of different reflection images (9) from the reflection pattern (8) reflected by the cornea (61) by virtue of the two-dimensional reference pattern (7) by colour filtering or polarization filtering; storage (S13) of the plurality of different reflection images (9);

**characterized by**

respective calculation of a phase value ($\varphi$) of the reflection pattern (8) for points (R) on the cornea (61) on the basis of intensities respectively measured in the stored reflection images (9) at a pixel (B) corresponding to the relevant point (R); and

determination (S2) of at least one measured geometrical value of the cornea (61) on the basis of the phase values ($\varphi$).

2. Method according to Claim 1, **characterized in that** a plurality of continuous, two-dimensional reference patterns (7) which have been phase-shifted with respect to one another are generated (S41) for the respective projection onto the cornea (61), and **in that** in each case a reflection image (9) of reflection patterns (8) reflected by the cornea (61) by virtue of the shifted reference pattern (7) is stored (S43).

3. Method according to Claim 2, **characterized in that** in each case a reflection image (9) of reflection patterns (8) reflected by the cornea (61) by virtue of the phase-shifted reference patterns (7) is stored (S43).

4. Method according to Claims 1, **characterized in that** the plurality of different reflection images (9) are produced (S52) from the same reflection pattern (8) reflected by the cornea (61) by virtue of a continuous, two-dimensional reference pattern (7), and stored (S53).

5. Method according to Claim 4, **characterized in that** the two-dimensional reference pattern (7) is generated (S51) from two mutually phase-shifted periodic basic reference patterns respectively having a different colour, and **in that** the reflection images (9) corresponding to the phase-shifted basic reference patterns (7) are produced (S52) by colour filtering from the reflection pattern (8).

6. Method according to Claim 4, **characterized in that** the two-dimensional reference pattern (7) is generated (S51) with pattern points (M) of different polarization, and **in that** the reflection images (9) are produced (S52) by polarization filtering from the reflection pattern.

7. Method according to one of Claims 1 to 6, **characterized in that** for points (R) on the cornea (61) the phase value ($\varphi$) of the reflection pattern (8) is respectively calculated on the basis of the reflection images (9) at the relevant point (R) by evaluating the equation

$$I(x',y') = I_0(x',y')\{1 + m(x',y')\cos[\varphi(x',y')]\},$$

$I(x',y')$ being a measured intensity at the corresponding pixel (B) with coordinates $x',y'$, $I_0(x',y')$ being a background intensity at the pixel (B), $m(x',y')$ being a signal modulation at the pixel (B), and $\varphi(x',y')$ being the phase value at the relevant point (R).

8. Method according to Claim 7, **characterized in that** two reference patterns (7) phase-shifted by 180° relative to one another are generated; **in that** two reflection images (9) of the reflection patterns (8) reflected by the cornea (61) by virtue of the two phase-shifted reference patterns (7) are stored (S62); and **in that** the background intensity and the signal modulation are calculated (S63) on the basis of the two reflection images (9) for the cornea (61).

9. Method according to Claim 8, **characterized in that** the background intensity is calculated on the basis of an addition of the two reflection images (9), and the signal modulation is calculated on the basis of a subtraction of the two reflection images (9).

10. Method according to one of Claims 1 to 9, **characterized in that** the reference patterns (7) are generated by modulation of at least one physical optical parameter from among light intensity, light polarization and light wavelength.

**11.** Method according to one of Claims 1 to 10, **characterized in that** a periodic reference pattern (7) is generated with a radially running and, for example, variable period.

**12.** Ophthalmological device (1) for measuring a cornea (61), comprising:

a reference pattern generator (31) set up to generate a two-dimensional reference pattern (7) from at least two mutually phase-shifted periodic basic reference patterns respectively having a different colour or pattern points (M) of different polarization;

a reference pattern system (3)set up to project the generated two-dimensional reference pattern (7) onto the cornea (61);

an image acquisition system (4) set up to acquire a reflection pattern (8) reflected by the cornea (61) by virtue of the reference pattern (7);

an extraction module (44) set up to generate a plurality of different reflection images (9) from the reflection pattern (8) reflected by the cornea (61) by virtue of the two-dimensional reference pattern (7) by colour filtering or polarization filtering;

wherein the image acquisition system (4) is set up to store the plurality of different reflection images (9);

**characterized by**

a processing module (2) set up to respectively calculate a phase value ($\varphi$) of the reflection pattern (8) for points (R) on the cornea (61), doing so on the basis of intensities respectively measured in the stored reflection images (9) at a pixel (B) corresponding to the relevant point (R), and determine the at least one measured geometrical value of the cornea (61) on the basis of the phase value ($\varphi$).

**13.** Device (1) according to Claim 12, **characterized in that** the reference pattern system (3) comprises an active display, in particular an LCD display, or a combination of a passive screen with a projector for the projection of the reference patterns (7) onto the screen.

**Revendications**

**1.** Procédé ophtalmologique de mesure d'une cornée (61), le procédé comportant les étapes qui consistent à :

former un motif bidimensionnel de référence (7) à l'aide d'un générateur (31) de motifs de référence à partir d'au moins deux motifs périodiques de référence de base, décalés l'un par rapport à l'autre par un déphasage et présentant chacun une couleur différente ou des points de motif (M) de polarisation différente,

projeter (S11) sur la cornée (61) le motif bidimensionnel de référence (7) ainsi formé,

saisir (S12) un motif de réflexion (8) réfléchi sur la cornée (61) sur la base du motif de référence (7),

former plusieurs images de réflexion (9) différentes à partir du motif de réflexion (8) réfléchi sur la cornée (61) sur la base du motif bidimensionnel de référence (7), par filtrage en fonction des couleurs ou filtrage polarisant,

conserver en mémoire (S13) les différentes images de réflexion (9),

**caractérisé par** les étapes qui consistent à

calculer pour des points (R) de la cornée (61) une valeur de phase ($\varphi$) du motif de réflexion (8) sur la base d'intensités mesurées sur un point d'image (B) qui correspond à un point concerné (R) dans les images de réflexion (9) conservées en mémoire et

déterminer (S2) au moins une valeur géométrique de mesure de la cornée (61) sur la base des valeurs de phase ($\varphi$).

**2.** Procédé selon la revendication 1, **caractérisé en ce que** plusieurs motifs bidimensionnels continus de référence (7), décalés les uns par rapport aux autres par un déphasage, sont formés (S41) pour être chaque fois projetés sur la cornée (61) et **en ce qu'**une image de réflexion (9) des motifs de réflexion (8) réfléchis par la cornée (61) sur la base des motifs de référence (7) décalés est conservée en mémoire (S43).

**3.** Procédé selon la revendication 2, **caractérisé en ce qu'**une image de réflexion (9) des différents motifs de réflexion (8) réfléchis par la cornée (61) sur la base des motifs de référence (7) déphasés est conservée en mémoire (S43).

**4.** Procédé selon la revendication 1, **caractérisé en ce que** les différentes images de réflexion (9) provenant du même motif de réflexion (8) réfléchi par la cornée (61) sur la base d'un motif de référence bidimensionnel continu (7) sont formées (S52) et conservées en mémoire (S53).

5. Procédé selon la revendication 4, **caractérisé en ce que** le motif bidimensionnel de référence (7) est formé (S51) à partir de deux motifs de référence de base déphasés l'un par rapport à l'autre et présentant chacun une couleur différente et **en ce que** les images de réflexion (9) qui correspondent aux motifs (7) de référence de base déphasés sont formés (S52) par filtrage du motif de réflexion (8) en fonction des couleurs.

6. Procédé selon la revendication 4, **caractérisé en ce que** le motif bidimensionnel de référence (7) est formé (S51) avec des points de motif (M) de polarisation différente et **en ce que** les images de réflexion (9) sont formées (S52) par filtrage polarisant du motif de réflexion.

7. Procédé selon l'une des revendications 1 à 6, **caractérisé en ce que** pour des points (R) de la cornée (61), sur la base des images de réflexion (9), la valeur de la phase ($\varphi$) du motif de réflexion (8) au point (R) concerné est calculée en évaluant l'équation :

$$I(x',y')=I_0(x',y')\{1+m(x',y')\cos[\varphi(x',y')]\}$$

dans laquelle *I (x',y')* est l'intensité mesurée sur le point d'image (B) correspondant de coordonnées *x',y', I_0 (x',y')* est l'intensité d'arrière-plan au point d'image (B), *m (x',y')* est une modulation du signal au point d'image (B) et $\varphi$*(x',y')* est la valeur de la phase au point (R) concerné.

8. Procédé selon la revendication 7, **caractérisé en ce que** deux motifs de référence (7) déphasés l'un par rapport à l'autre de 180° sont formés (S61), **en ce que** deux images de réflexion (9) des motifs de réflexion (8) réfléchis par la cornée (61) sur la base des deux motifs de référence (7) déphasés sont conservées en mémoire (S62) et **en ce que** l'intensité d'arrière-plan et la modulation des signaux sont calculées (S63) sur la base des deux images de réflexion (9) de la cornée (61).

9. Procédé selon la revendication 8, **caractérisé en ce que** l'intensité d'arrière-plan est calculée sur la base de l'addition de deux images de réflexion (9) et **en ce que** la modulation des signaux est calculée sur la base d'une soustraction des deux images de réflexion (9).

10. Procédé selon l'une des revendications 1 à 9, **caractérisé en ce que** les motifs de référence (7) sont formés par modulation d'au moins un paramètre physique de la lumière sélectionné parmi l'intensité de la lumière, la polarisation de la lumière et la longueur d'onde de la lumière.

11. Procédé selon l'une des revendications 1 à 10, **caractérisé en ce qu'**un motif périodique de référence (7) présentant une période qui s'étend radialement et qui peut par exemple être modifiée est formé.

12. Ensemble ophtalmologique (1) destiné à mesurer une cornée (61), l'ensemble comprenant :

un générateur (31) de motifs de référence conçu pour former un motif bidimensionnel de référence (7) à partir d'au moins deux motifs périodiques de référence de base déphasés l'un par rapport à l'autre par un déphasage, et présentant chacun une couleur différente ou des points de motif (M) de polarisation différente,
un système (3) de motifs de référence conçu pour projeter sur la cornée (61) le motif bidimensionnel de référence (7) ainsi formé,
un système (4) de saisie d'image conçu pour saisir un motif de réflexion (8) réfléchi par la cornée (61) sur la base du motif de référence (7),
un module d'extraction (44) conçu pour former plusieurs images de réflexion (9) différentes à partir des motifs de réflexion (8) réfléchis par la cornée (61) sur la base du motif bidimensionnel de référence (7), par filtrage en fonction des couleurs ou filtrage polarisant,
le système (4) de saisie d'image étant conçu pour conserver en mémoire les différentes images de réflexion (9),
**caractérisé par**
un module de traitement (2) conçu pour calculer pour des points (R) de la cornée (61) une valeur de phase ($\varphi$) du motif de réflexion (8) sur la base d'intensités mesurées dans les images de réflexion (9) conservées en mémoire sur un point d'image (B) qui correspond au point concerné (R) et pour déterminer au moins une valeur de mesure géométrique de la cornée (61) sur la base de la valeur de la phase ($\varphi$).

13. Ensemble (1) selon la revendication 12, **caractérisé en ce que** le système (3) de motifs de référence comporte un affichage actif, en particulier un affichage à LCD ou une combinaison d'un écran passif et d'un projecteur qui projette le motif de référence (7) sur l'écran.

**Fig. 1**

S11 — ERZEUGEN UND PROJIZIEREN VON MINDESTENS EINEM REFERENZMUSTER

S12 — ERFASSEN VON MINDESTENS EINEM REFLEXIONSMUSTER

S13 — SPEICHERN VON MEHREREN REFLEXIONSBILDERN

S1

S2 — BESTIMMEN EINES GEOMETRISCHEN MESSWERTS BASIEREND AUF DEN MEHREREN REFLEXIONSBILDERN

S3 — AUSGEBEN VON MESSRESULTAT(EN)

**Fig. 2**

S41 → ERZEUGEN UND PROJIZIEREN VON MEHREREN ZUEINANDER VERSCHOBENEN REFERENZMUSTERN

S42 → ERFASSEN VON MEHREREN AUF GRUND DER REFERENZMUSTER REFLEKTIERTEN REFLEXIONSMUSTERN

S4

S43 → SPEICHERN VON MEHREREN DEN REFLEXIONSMUSTERN ENTSPRECHENDEN REFLEXIONSBILDERN

S2 → BESTIMMEN EINES GEOMETRISCHEN MESSWERTS BASIEREND AUF DEN MEHREREN REFLEXIONSBILDERN

S3 → AUSGEBEN VON MESSRESULTAT(EN)

# Fig. 3

```
┌ ─ ─ ─ ─ ─ ─ ─ ─ ─ ─ ─ ─ ─ ─ ─ ─ ┐
│      ┌─────────────────────┐      │
│      │   ERZEUGEN UND      │      │
│      │  PROJIZIEREN VON    │      │
│      │       EINEM         │      │
│      │  REFERENZMUSTER     │      │
│      └─────────────────────┘      │
```

S51

```
│      ┌─────────────────────┐      │
│      │   ERZEUGEN VON      │      │
│      │    MEHREREN         │      │
│      │  REFLEXIONSBILDERN  │      │
│      │  AUS EINEM AUF      │      │
```

S52

```
│      │   GRUND DES         │      │
│      │  REFERENZMUSTERS    │      │
│      │   REFLEKTIERTEN     │      │
│      │  REFLEXIONSMUSTER   │      │
│      └─────────────────────┘      │
```

S5

```
│      ┌─────────────────────┐      │
│      │   SPEICHERN VON     │      │
│      │  MEHREREN AUS DEM   │      │
│      │  REFLEXIONSMUSTER   │      │
│      │    ERZEUGTEN        │      │
```

S53

```
│      │  REFLEXIONSBILDERN  │      │
│      └─────────────────────┘      │
└ ─ ─ ─ ─ ─ ─ ─ ─ ─ ─ ─ ─ ─ ─ ─ ─ ┘
```

```
       ┌─────────────────────┐
       │  BESTIMMEN EINES    │
       │  GEOMETRISCHEN      │
       │   MESSWERTS         │
       │  BASIEREND AUF      │
       │  DEN MEHREREN       │
       │  REFLEXIONSBILDERN  │
       └─────────────────────┘
```

S2

```
       ┌─────────────────────┐
       │  AUSGEBEN VON       │
       │  MESSRESULTAT(EN)   │
       └─────────────────────┘
```

S3

# Fig. 4

S61 → ERZEUGEN UND PROJIZIEREN EINES REFERENZMUSTERS AUS ZWEI ZUEINANDER UM 180° PHASENVERSCHOBENEN REFERENZGRUNDMUSTERN

S62 → ERZEUGEN UND SPEICHERN VON ZWEI REFLEXIONSBILDERN AUS DEM AUF GRUND DES REFERENZMUSTERS REFLEKTIERTEN REFLEXIONSMUSTER

S63 → BERECHNEN VON HINTERGRUNDINTENSITÄT UND SIGNALMODULATION BASIEREND AUF DEN ZWEI REFLEXIONSBILDERN

S6

S41' → ERZEUGEN UND PROJIZIEREN VON EINEM WEITEREN REFERENZMUSTER

S42' → ERFASSEN VON EINEM AUF GRUND DES WEITEREN REFERENZMUSTERS REFLEKTIERTEN WEITEREN REFLEXIONSMUSTER

S43' → SPEICHERN VON EINEM WEITEREN DEM WEITEREN REFLEXIONSMUSTER ENTSPRECHENDEN REFLEXIONSBILD

S4'

S2' → BESTIMMEN EINES GEOMETRISCHEN MESSWERTS BASIEREND AUF DER HINTER-GRUNDINTENSITÄT, DER SIGNALMODULATION UND DEM WEITEREN REFLEXIONSBILD

S3 → AUSGEBEN VON MESSRESULTAT(EN)

# Fig. 5

S61 — ERZEUGEN UND PROJIZIEREN EINES REFERENZMUSTERS AUS ZWEI ZUEINANDER UM 180° PHASENVERSCHOBENEN REFERENZGRUNDMUSTERN

S62 — ERZEUGEN UND SPEICHERN VON ZWEI REFLEXIONSBILDERN AUS DEM AUF GRUND DES REFERENZMUSTERS REFLEKTIERTEN REFLEXIONSMUSTER

S63 — BERECHNEN VON HINTERGRUNDINTENSITÄT UND SIGNALMODULATION BASIEREND AUF DEN ZWEI REFLEXIONSBILDERN

S6

S51' — ERZEUGEN UND PROJIZIEREN VON EINEM WEITEREN REFERENZMUSTER

S52' — ERZEUGEN VON MEHREREN WEITEREN REFLEXIONSBILDERN AUS EINEM AUF GRUND DES WEITEREN REFERENZMUSTERS REFLEKTIERTEN REFLEXIONSMUSTER

S53' — SPEICHERN VON MEHREREN AUS DEM REFLEXIONSMUSTER ERZEUGTEN WEITEREN REFLEXIONSBILDERN

S5'

S2" — BESTIMMEN EINES GEOMETRISCHEN MESSWERTS BASIEREND AUF DER HINTER-GRUNDINTENSITÄT, DER SIGNALMODULATION UND DEN WEITEREN REFLEXIONSBILDERN

S3 — AUSGEBEN VON MESSRESULTAT(EN)

# Fig. 6

**Fig. 7a**

**Fig. 7b**

**Fig. 7c**

**Fig. 7d**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5953100 A
- US 6926408 B
- US 5116115 A
- US 5592246 A
- DE 102007018048